# EUROPEAN PATENT APPLICATION

(11) **EP 4 588 440 A1**
(43) Date of publication of application: **23.07.2025**
(21) Application number: 23865890.0
(22) Date of filing: 15.09.2023
(51) Int. Cl.: A61B 5/293, A61B 5/0536

(54) **NASAL CAVITY INSERTION-TYPE ELECTRODE DEVICE FOR NERVE DETECTION AND IMAGE CAPTURING**

(30) Priority: 16.09.2022 KR 20220116979
(71) Applicant: Liin Technologies Incorporated, Newport News, Virginia 23606 (US); Space Liintech Co., Ltd., Daejeon 34054 (KR)
(72) Inventor: YOON, Hargsoon, Yorktown, Virginia 23693 (US)
(74) Representative: Modiano, Gabriella Diana
(86) International application number: PCT/KR2023/013883
(87) International publication number: WO 2024/058594

(57) **Abstract**

The present invention relates to an electrode device configured to enable an electrode to be inserted into a desired position in the nasal cavity. A nasal cavity insertion-type electrode device according to an embodiment of the present invention includes: an electrode part which includes a first end having a fine hole formed therethrough and a second end having an open part formed therethrough to allow a fluid to enter or exit, and has an electrode disposed therein; and a guide part including a third end formed to be expandable and a fourth end having an open part formed therethrough to allow a fluid to enter or exit, wherein the first end and the third end are arranged adjacent to each other.

## Description

### TECHNICAL FIELD

The following description relates to a nasal cavity insertion-type electrode device, and more particularly, to an electrode device configured to enable insertion of an electrode into a desired position within the nasal cavity.

### BACKGROUND ART

Existing measurement methods for brain activity include electroencephalography (EEG) and magnetoencephalography (MEG) technologies that measure electrical activity of brain nerves. In addition, there are electrical impedance tomography (EIT) that measures structural changes in the brain and electrical contact means for electrical stimulation of the brain.

The electrical impedance tomography technology is a technology that visualizes the impedance, electrical conductivity, or permittivity of a living body by applying current through electrodes attached to the surface of the body and then measuring the voltage through other electrodes attached to the surface.

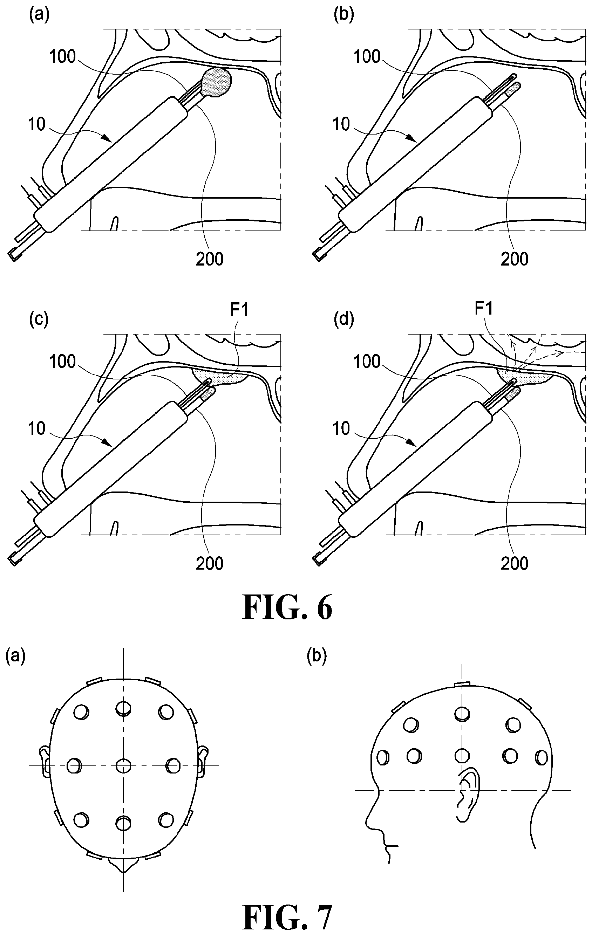

In the above-described methods, sensor or electrode is positioned on a scalp above a skull, as illustrated in FIG. 7, to measure brain activity or status. Typically, electrodes made of metal or ceramic are attached to the scalp using an electrically conductive adhesive, or fixed on an elastic bandana covering the head, and then electrically contacted with the scalp through a conductive gel. However, such conventional electrode placement has limitations in that it distorts signals due to the low electrical conductivity of the skull or makes it difficult to accurately image the brain.

Recently, technologies have been developed to measure internal radiation dose in real time during radiation therapy using the electrical impedance tomography technology, and to monitor the degree and position of brain tissue and signal response to radiation exposure. In addition, recent brain research deals with higher-order cognitive functions such as memory and emotion, and in order to deal with these topics, it is necessary to measure signals from deep brain structures such as the thalamus and hippocampus.

### DISCLOSURE OF THE INVENTION

### TECHNICAL GOALS

Accordingly, in the nasal cavity insertion-type electrode device of the present disclosure, an aspect thereof is to provide a nasal cavity insertion-type electrode device that measures electroencephalogram signals or electrical impedance signals in parallel with electrodes positioned on a skull.

In addition, in the nasal cavity insertion-type electrode device of the present disclosure, an aspect thereof is to provide a nasal cavity insertion-type electrode device that enables an electrode to be placed at a desired position without damaging tissues and nerve cells within a nasal cavity.

### TECHNICAL SOLUTIONS

According to one embodiment of the present disclosure for achieving the above-described aspects, a nasal cavity insertion-type electrode device includes: an electrode part which includes a first end having a fine hole formed therethrough and a second end having an open part formed therethrough to allow a fluid to enter or exit, and has an electrode disposed therein; and a guide part including a third end formed to be expandable and a fourth end having an open part formed therethrough to allow a fluid to enter or exit, in which the first end and the third end are arranged adjacent to each other.

In addition, in the nasal cavity insertion-type electrode device of the present disclosure, the electrode part may be formed as a tube that has a predetermined diameter and is bent so that the first end has a U shape, and the electrode may be inserted through the second end and disposed adjacent to the fine hole.

In addition, in the nasal cavity insertion-type electrode device of the present disclosure, the second end may include a first open part and a second open part, which are both open parts of the tube, and the fluid may flow in from the first open part and flow out from the second open part, and at least a portion of the fluid may be discharged through the fine hole by a pressure difference between the first open part and the second open part.

Moreover, in the nasal cavity insertion-type electrode device of the present disclosure, the electrode part may include at least one electrode, and the electrode may be inserted from a first electrode entry/exit part branched from the first open part or a second electrode entry/exit part branched from the second open part.

Furthermore, in the nasal cavity insertion-type electrode device of the present disclosure, the guide part may be formed as a tube that has a predetermined diameter and is bent so that the third end has a U shape, and at least a portion of the third end may be formed as an expandable member.

In addition, in the nasal cavity insertion-type electrode device of the present disclosure, the fourth end may include a third open part and a fourth open part which are both open parts of the tube, the third open part may be sealed with a sealing member, and the fluid flows in from the fourth open part to expand the expandable member.

In addition, the nasal cavity insertion-type electrode device of the present disclosure may further include a body part that fixes relative positions of the electrode part and the guide part.

Moreover, in the nasal cavity insertion-type electrode device of the present disclosure, the electrode part and the guide part may be disposed so that the third end surrounds the first end when the third end is expanded.

### EFFECTS OF THE INVENTION

According to the nasal cavity insertion-type electrode device of the present disclosure, electrode(s) can be placed close to the skull, such as the cribriform plate of ethmoide bone, within the nasal cavity, and using the electrode(s), electroencephalogram signals or electrical impedance signals can be measured, and a necessary amount of current can flow into the brain tissue through the cribriform plate of ethmoid bone.

In addition, by using the nasal cavity insertion-type electrode device of the present disclosure, signals of deep brain structures can be measured more accurately in parallel with electrodes disposed on the scalp, and the precision of brain imaging can be improved.

In addition, according to the nasal cavity insertion-type electrode device of the present disclosure, the electrode can be safely placed in a desired position without damaging tissues within the nasal cavity or maintaining the performance of the electrode itself.

### BRIEF DESCRIPTION OF THE DRAWINGS

FIG. 1 is a view for explaining an exemplary arrangement state of a nasal cavity insertion-type electrode device according to one embodiment of the present disclosure.
FIG. 2 is a view schematically illustrating the configuration of the nasal cavity insertion-type electrode device according to one embodiment of the present disclosure.
FIG. 3 is a view illustrating a guide part of the nasal cavity insertion-type electrode device of FIG. 2 in an expanded state.
FIG. 4 is a view specifically illustrating the configuration of an electrode part of the nasal cavity insertion-type electrode device of FIG. 2.
FIG. 5 is a view specifically illustrating the configuration of the guide part of the nasal cavity insertion-type electrode device of FIG. 2.
FIG. 6 is a view for explaining an exemplary usage state of the nasal cavity insertion-type electrode device according to one embodiment of the present disclosure.
FIG. 7 is a view for explaining a conventional electrode device positioned on a scalp.

### BEST MODE FOR CARRYING OUT THE INVENTION

The advantages and features of the present disclosure, and the methods for achieving them, will become clear with reference to embodiments described in detail below together with the accompanying drawings. However, the present disclosure is not limited to the embodiments disclosed below, but may be implemented in various different forms, and these embodiments are provided only to make the disclosure of the present disclosure complete and to fully inform a person having ordinary skill in the art to which the present disclosure belongs of the scope of the disclosure, and the present disclosure is defined only by the scope of the claims.

Although the terms first, second, or the like are used to describe various components, it is to be understood that these components are not limited by these terms. These terms are merely used to distinguish one component from another. Accordingly, it is to be understood that a first component referred to below may also be a second component within the technical concept of the present disclosure.

In the embodiments below, terms such as "include" or "have" mean that a feature or component described in the specification is present, and do not exclude in advance the possibility that one or more other features or components may be added.

In the drawings, the sizes of components may be exaggerated or reduced for convenience of explanation. For example, the sizes and shapes of each component illustrated in the drawings are arbitrarily illustrated for convenience of explanation, and therefore the present disclosure is not necessarily limited to what is illustrated.

Throughout the specification, identical reference numerals refer to identical components.

The individual features of the various embodiments of the present disclosure may be partially or wholly combined or combined with each other, and as may be fully understood by those skilled in the art, various technical connections and operations are possible, and each embodiment may be implemented independently of each other or may be implemented together in a related relationship.

Hereinafter, a nasal cavity insertion-type electrode device of the present disclosure will be described in detail with reference to the attached drawings.

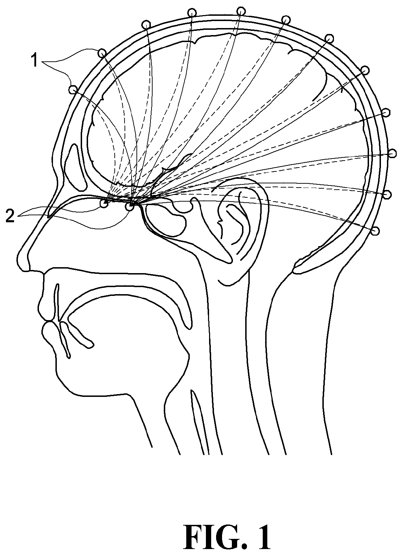

FIG. 1 is a view for explaining an exemplary arrangement state of a nasal cavity insertion-type electrode device according to one embodiment of the present disclosure.

Referring to FIG. 1, a nasal cavity insertion-type electrode device 2 of the present disclosure may measure electroencephalogram signals or electrical impedance signals in parallel with a scalp electrode 1 positioned on the skull.

Desirably, the nasal cavity insertion-type electrode device 2 of the present disclosure may be positioned on a mucosal surface of a nasal cavity. For example, the nasal cavity insertion-type electrode device 2 of the present disclosure may be positioned close to the cribriform plate of ethmoide bone. The skull between the nasal cavity and the brain has the cribriform plate of the ethmoid bone, which is a perforated structure for connecting the olfactory nerve, so when the electrode is positioned in the cribriform plate of ethmoid bone, there is an advantage in that a signal may be measured with high conductivity.

The nasal cavity insertion-type electrode device 2 of the present disclosure may be inserted into both sides of the nose and positioned as a pair within the nasal cavity as illustrated in FIG. 1.

However, the positions and numbers illustrated in FIG. 1 are exemplary only, and the scope of the present disclosure is not limited thereto.

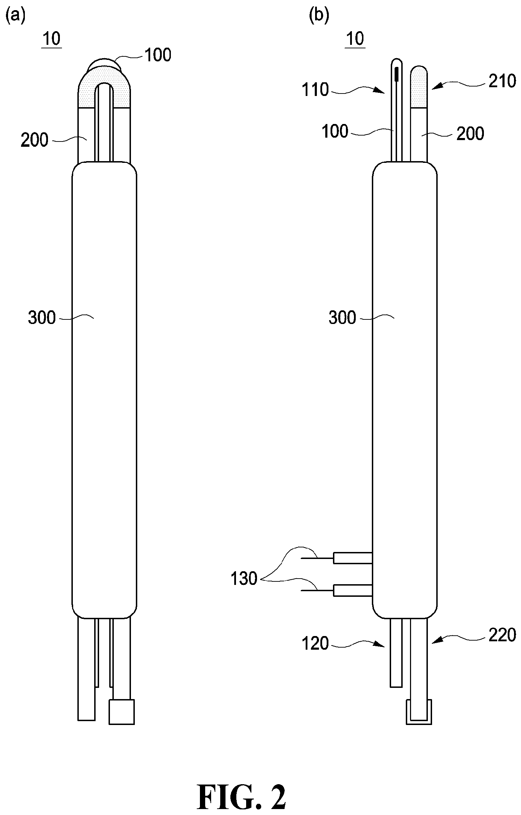

FIG. 2 is a view schematically illustrating the configuration of the nasal cavity insertion-type electrode device 10 according to one embodiment of the present disclosure.

(a) of FIG. 2 illustrates a front view of the electrode device 10, and (b) of FIG. 2 illustrates a side view of the electrode device 10.

Referring to FIG. 2, the nasal cavity insertion-type electrode device 10 according to one embodiment of the present disclosure includes an electrode part 100 in which an electrode 130 is disposed therein, a guide part 200 disposed adjacent to the electrode part 100, and a body part 300 that fixes the relative positions of the electrode part 100 and the guide part 200.

The electrode part 100 includes a first end 110 having a fine hole formed therethrough and a second end 120 having an open part formed therethrough to allow a fluid to enter and exit, and the electrode 130 is disposed inside the electrode part 100. The guide part 200 includes a third end 210 formed to be expandable and a fourth end 220 having open part formed therethrough to allow the fluid to enter and exit. In this case, the first end 110 of the electrode part 100 and the third end 210 of the guide part 200 are disposed adjacent to each other.

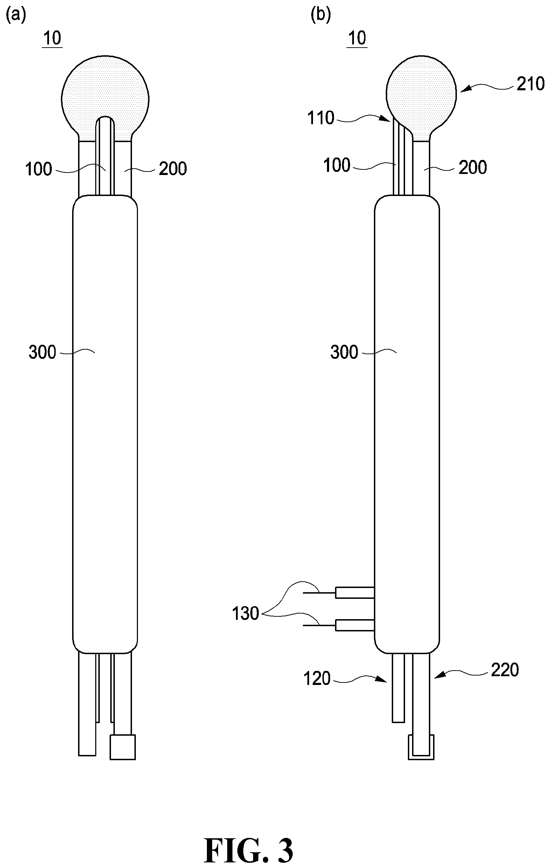

In order to specifically explain each configuration of the nasal cavity insertion-type electrode device 10 according to one embodiment of the present disclosure, reference will be made to FIGS. 3 to 5 below.

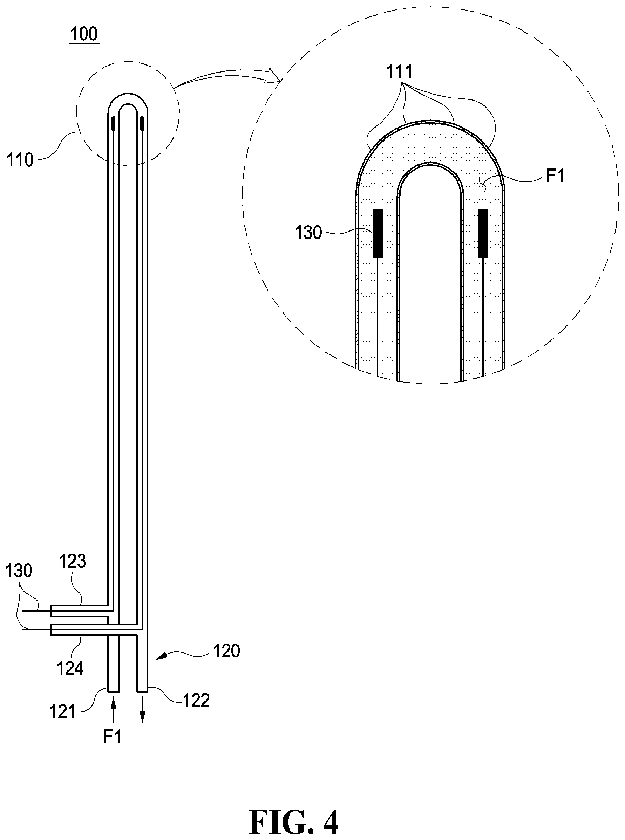

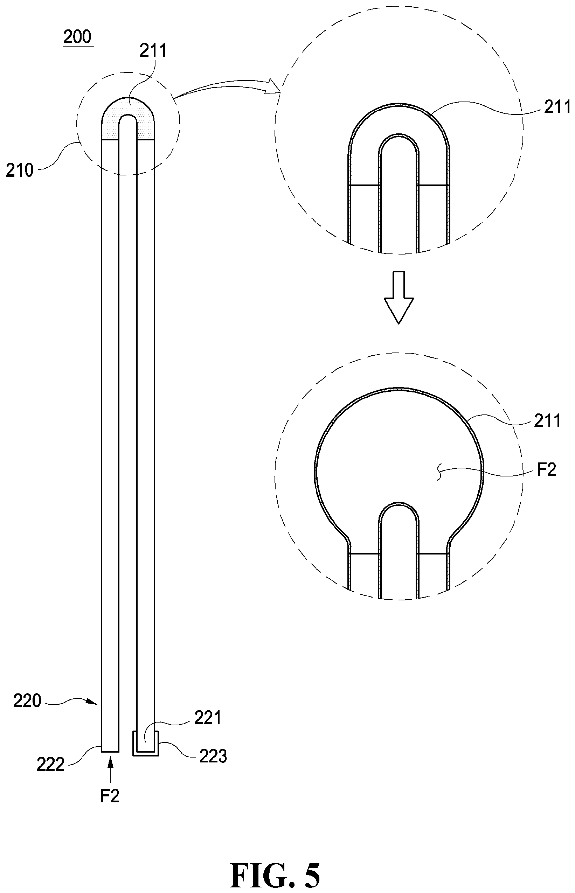

FIG. 3 is a view illustrating a portion of the guide part 200 of the nasal cavity insertion-type electrode device 10 of FIG. 2 in an expanded state, FIG. 4 is a view specifically illustrating the configuration of the electrode part 100 of the nasal cavity insertion-type electrode device 10 of FIG. 2, and FIG. 5 is a view specifically illustrating the configuration of the guide part 200 of the nasal cavity insertion-type electrode device 10 of FIG. 2.

Referring to FIGS. 2 and 4, the electrode part 100 is formed as a tube that has a predetermined diameter and is bent so that the first end 110 has a U shape. The electrode 130 disposed inside the electrode part 100 is inserted through the second end 120 and disposed adjacent to a fine hole 111 formed on the side of the first end 110.

The fine hole 111 may be in the form of at least a portion of the tube of the electrode part 100 penetrating. At least one fine hole 111 may be formed, and desirably two or more may be formed.

The second end 120 of the electrode part 100 includes a first open part 121 and a second open part 122, which are both open parts of the tube. In addition, the second end 120 may include a first electrode entry/exit part 123 branched from the first open part 121 and a second electrode entry/exit part 124 branched from the second open part 122.

A fluid F1 flows through the inside of the electrode part 100, and the electrode 130 may be electrically connected to an electrode installed at another position by the fluid F1 flowing inside. This will be described in detail with reference to FIG.6 described below.

The fluid F1 may flow in from the first open part 121 and flow out from the second open part 122, as illustrated in FIG. 4. In this case, at least a portion of the fluid F1 may be discharged through the fine hole 111 formed in the first end 110 due to the pressure difference between the first open part 121 and the second open part 122.

In this case, the fluid F1 may be a liquid, such as phosphate-buffered saline (PBS) or artificial cerebrospinal fluid (aCSF), for example. In addition, the fluid F1 may be a gas. Various other fluids may be applied to the fluid F1, and the fluid F1 is not specifically limited in the present disclosure.

The electrode part 100 may include two electrodes 130 as illustrated. The electrodes 130 may be inserted from the first electrode entry/exit part 123 branched from the first open part 121 and the second electrode entry/exit part 124 branched from the second open part 122, respectively. For example, an insulator-coated fine metal wire (tungsten, stainless steel, gold, iridium, platinum), or the like may be used as the electrode 130, and like Ag/AgCl electrodes and Iridium Oxide electrodes, microelectrodes that have improved performance through surface treatment and coating processes to discharge high-density charges and are friendly to living bodies may be used.

Referring to FIGS. 3 and 5, the guide part 200 is formed as a tube that has a predetermined diameter and is bent so that the third end 210 has a U shape. At least a portion of the third end 210 is formed of an expandable member 211.

The fourth end 220 of the guide part 200 includes a third open part 221 and a fourth open part 222, which are both open parts of the tube.

The third open part 221 may be sealed with a sealing member 223, and a fluid F2 may flow in from the fourth open part 222 to expand the expansion member 211 of the third end 210. Here, the expansion member 211 may be formed of an elastic material such as rubber and may be inflated like a balloon by the fluid F2 flowing inside the guide part 200.

As illustrated in FIG. 3, when the expansion member 211 of the third end 210 of the guide part 200 expands, the third end 210 surrounds the first end 110 of the electrode part 100. Since the expanded third end 210 surrounds the first end 110, the nasal cavity insertion-type electrode device 10 of the present disclosure may be safely placed in the nasal cavity without damaging the tissues in the nasal cavity. In particular, the first end 110 where the electrode 130 is disposed may be safely placed in a desired position without being damaged by the tissues in the nasal cavity.

Meanwhile, the body part 300 fixes the relative positions of the electrode part 100 and the guide part 200. Specifically, the body part 300 may fix the relative positions of the electrode part 100 and the guide part 200 so that the first end 110 of the electrode part 100 and the third end 210 of the guide part 200 are disposed to each other. More specifically, the body part 300 may fix the relative positions of the electrode part 100 and the guide part 200 so that when the expansion member 211 of the third end 210 is expanded, the third end 210 may surround the first end 110.

FIG. 6 is a view for explaining an exemplary usage state of the nasal cavity insertion-type electrode device according to one embodiment of the present disclosure.

Referring to FIG. 6 below, a process in which the electrode part 100 and the guide part 200 operate during the process of inserting the nasal cavity insertion-type electrode device 10 of the present disclosure into the nasal cavity will be briefly described.

(a) of FIG. 6 illustrates a state in which the nasal cavity insertion-type electrode device 10 of the present disclosure is inserted into the nasal cavity in a state where the expansion member 211 of the guide part 200 expanded. The expansion member 211 is inflated like a balloon by the fluid F2 flowing inside the guide part 200. The expansion member 211 surrounds the first end 110 of the electrode part 100, and the first end 110 where the electrode 130 is disposed may be safely placed in a preset position without damaging the tissues in the nasal cavity or being damaged by the tissues in the nasal cavity.

In this case, the preset position may be the cribriform plate of ethmoid bone, which is a portion of the skull between the nasal cavity and the brain, as illustrated.

When the nasal cavity insertion-type electrode device 10 of the present disclosure is inserted into the preset position, the expansion member 211 of the guide part 200 may come into contact with the mucous membrane in the nasal cavity. For example, the entry of the nasal cavity insertion-type electrode device 10 may be terminated by this signal.

(b) of FIG. 6 illustrates a state in which the expansion member 211 of the guide part 200 is restored to an original state of the expansion member after the nasal cavity insertion-type electrode device 10 of the present disclosure is placed at a desired position. In other words, the pressure of the fluid flowing into the inside of the guide part 200 is lowered, and the expansion member 211 is illustrated to have contracted to the original state. In this case, the first end 110 of the electrode part 100 where the electrode 130 is disposed may be exposed again.

(c) of FIG. 6 illustrates a state in which the fluid F1 is discharged from the electrode part 100. As described above, the fluid F1 flows inside the electrode part 100, and at least a portion of the fluid F1 may be discharged through the fine holes 111 due to the pressure difference between the open parts open on both sides of the electrode part 100.

As illustrated in (d) of FIG. 6, the fluid F1 induces a desirable electrical contact between the electrode 130 positioned inside the electrode part 100 and the tissue (for example, cribriform plate of ethmoid bone) inside the nasal cavity, and the electrode 130 inside the electrode part 100 may be electrically connected to an electrode installed in another position by the fluid F1.

Through this process, the nasal cavity insertion-type electrode device 10 of the present disclosure may place an electrode for measuring an electroencephalogram signal or an electrical impedance signal inside the nasal cavity, and in parallel with other electrodes disposed on the scalp, may more accurately measure signals of deep brain structures or increase the precision of brain imaging.

Although the embodiments of the present disclosure have been described with reference to the attached drawings, those skilled in the art will understand that the present disclosure may be implemented in other specific forms without changing the technical idea or essential features of the present disclosure. Therefore, it should be understood that the embodiments described above are exemplary in all respects and not restrictive.

## Claims

1. A nasal cavity insertion-type electrode device comprising:
an electrode part which comprises a first end having a fine hole formed therethrough and a second end having an open part formed therethrough to allow a fluid to enter or exit, and has an electrode disposed therein; and
a guide part comprising a third end formed to be expandable and a fourth end having an open part formed therethrough to allow a fluid to enter or exit,
wherein the first end and the third end are arranged adjacent to each other.

2. The nasal cavity insertion-type electrode device according to claim 1, wherein
the electrode part is formed as a tube bent so that the first end has a U shape, and
the electrode is inserted through the second end and disposed adjacent to the fine hole.

3. The nasal cavity insertion-type electrode device according to claim 2, wherein
the second end comprises a first open part and a second open part, which are both open parts of the tube, and
the fluid flows in from the first open part and flows out from the second open part, and at least a portion of the fluid is discharged through the fine hole by a pressure difference between the first open part and the second open part.

4. The nasal cavity insertion-type electrode device according to claim 3, wherein
the electrode part comprises at least one electrode, and
the electrode is inserted from a first electrode entry/exit part branched from the first open part or a second electrode entry/exit part branched from the second open part.

5. The nasal cavity insertion-type electrode device according to claim 1, wherein
the guide part is formed as a tube bent so that the third end has a U shape, and
the third end is formed as an expandable member.

6. The nasal cavity insertion-type electrode device according to claim 5, wherein
the fourth end comprises a third open part and a fourth open part which are both open parts of the tube,
the third open part is sealed with a sealing member, and
the fluid flows in from the fourth open part to expand the expandable member.

7. The nasal cavity insertion-type electrode device according to claim 1, further comprising:
a body part that fixes positions of the electrode part and the guide part.

8. The nasal cavity insertion-type electrode device according to claim 1, wherein
the electrode part and the guide part are disposed so that the third end surrounds the first end when the third end is expanded.
